# EUROPEAN PATENT SPECIFICATION

(11) **EP 1 023 467 B1**
(45) Date of publication and mention of the grant of the patent: **03.03.2004**
(21) Application number: 97932541.2
(22) Date of filing: 07.07.1997
(51) Int. Cl.: C12Q 1/70, G01N 33/53, C12N 15/00, C12N 7/00

(54) **METHOD FOR MEASURING VIRAL INFECTIVITY**
VERFAHREN ZUR MESSUNG VIRALER INFEKTIVITÄT
PROCEDE POUR MESURER L'INFECTIOSITE VIRALE

(30) Priority: 09.07.1996 US 678485
(43) Date of publication of application: 02.08.2000
(73) Proprietor: CANJI, Inc., San Diego, CA 92121 (US)
(72) Inventor: HUTCHINS, Beth, M., San Diego, CA 92129 (US); NUNNALLY, Mary, H., Corrales, NM 87048 (US); SUGARMAN, Barry, J., San Diego, CA 92129 (US)
(74) Representative: UEXKÜLL & STOLBERG
(86) International application number: PCT/US1997/011865
(87) International publication number: WO 1998/001582

(56) References cited:
- EP-A- 0 697 414
- US-A- 5 470 730
- DEMARCHI J.M. ET AL: "Expression of the genome of noninfectious particles in stocks of standard and defective, interfering pseudorabies virions." VIROLOGY, (1979) 97/2 (457-463). CODEN: VIRLAX, XP001022716
- HUYGHE B.G. ET AL: "Purification of a type 5 recombinant adenovirus encoding human p53 by column chromatography." HUMAN GENE THERAPY, (1995) 6/11 (1403-1416)., XP000196636
- ROHOZINSKI J ET AL: "Infectivity of algal viruses studied by chlorophyll fluorescence." JOURNAL OF GENERAL VIROLOGY, vol. 76, no. 11, 1995, pages 2859-2862, XP001033793 ISSN: 0022-1317
- OLKKONEN V M ET AL: "QUANTITATION OF THE ADSORPTION AND PENETRATION STAGES OF BACTERIOPHAGE PHI-6 INFECTION" VIROLOGY, vol. 171, no. 1, 1989, pages 229-238, XP001018811 ISSN: 0042-6822
- J. VIR. METH., 1993, Vol. 44, SAALMULLER A., "Rapid Identification and Quantification of Cells Infected by Recombinant Herpesvirus (Pseodirabies Virus) Using a Fluorescence-Based B-Galactosidase Assay and Flow Cytometry", pages 99-108, XP002909835.
- J. VIR. METH., 1994, Vol. 46, EYLER Y.L., "Flow Cytometric Detection of DNA Tumor Virus Nuclear Oncogene Products in Unfixed Cells: Saponin FACS of Viral Oncogene Products", pages 23-27, XP002909836.
- J. IMMUN. METH., 1994, Vol. 169, WEN S.F., "Retinoblastoma Protein Monoclonal Antibodies with Novel Characteristics", pages 231-240, XP002909826.

## Description

### BACKGROUND OF THE INVENTION

A particular challenge in the delivery of a gene by a viral vector for therapeutic purposes is the preparation and accurate characterization of clinical dosage forms. Total particle measurement can be made by such techniques as electron microscopy of viral preparations or measurement of total DNA by optical density at 260 nm of a sodium dodecyl sulfate (SDS) treated virus suspension. However, infectivity of a viral preparation, i.e., the number of infectious viral particles in a preparation of virus, is more challenging to accurately measure.

Traditionally, infectivity particles are measured in culture by a plaque-forming unit assay (pfu) that scores the number of viral plaques as a function of dilution. An alternative to the pfu assay is the tissue culture infective dose procedure (TCID₅₀), which estimates infectivity as a function of intracellular staining for an antigen by direct immunofluorescence. The methods suffer from limitations including a high degree of inter-assay variability and are affected by such factors as virus replication status, vector characteristics, and virus-cell interactions.

More recently, flow cytometry or FACS (fluorescence-activated cell sorter) assays have been used to measure the number of infected cells in cell cultures infected at relatively high multiplicities of infection. For example, Saalmüller and Mettenleiter (J. Virol. Methods **44:99-108** (1993)) disclose the identification and quantitation of cells infected by recombinant pseudorabies virus mutants by the reaction of intracellular β-galactosidase expressed during infection with recombinant viruses with a fluorogenic substrate, followed by detection of positive cells in flow cytometry. Morris et al. (Virology 197(1):**339-48** (1993)) studied the process of productive and non-productive recombinant AcMNPV infection in cultured cells by immunostaining cells to detect the reporter CAT gene product.

The instant invention addresses the need for a more accurate method of quantitating infectious viral particles in a population.

### SUMMARY OF THE INVENTION

The methods of the instant invention are based on the unexpected and surprising result that flow cytometry analysis of cells infected at a low virus to cell ratio yields a more accurate measurement of infectious virus titer than traditional titration methods.

The invention is a method for determining the number of infectious virus particles in a population of virus particles comprising:
i) infecting cells in a cell population at a total particle to cell ratio of less than about 100:1 to about 0.1:1 to generate infected cells;
ii) reacting a polypeptide expressed by the virus in infected cells with an antibody labeled with a fluorescent tag, the antibody having specificity for a polypeptide expressed by the virus; and
iii) measuring immunofluorescence in the product of step (ii) by flow cytometry to determine the number of infected cells, thereby determining the number of infectious virus particles.

When the virus is a recombinant virus, the viral polypeptide can be encoded by an exogenous gene, such as a reporter gene. In some embodiments of the invention, the exogenous gene is a tumor suppressor gene such as p53 or retinoblastoma (RB). The recombinant virus can be replication competent or defective, deficient or incompetent.

In some embodiments of the invention, the virus is adenovirus. Thus, when the infected cells are cultured after infection to allow expression of a viral polypeptide, the viral polypeptide can be an adenovirus polypeptide such as hexon.

The viral polypeptide is reacted with at least one antibody, although the antibody can be a mixture of antibodies. The antibody can be polyclonal or monoclonal.

The total particle to cell ratio is less than about 100:1, typically less than about 10:1, preferably less than about 5:1, more preferably less than about 1:1. In some embodiments, the ratio can be as low as about 0.1:1.

### DETAILED DESCRIPTION

The instant invention provided methods for quantitating infectious viral particles in a population of virus particles. The term "infectious" as used herein is intended to refer to the ability of a virus to enter cells and direct the synthesis of at least one polypeptide encoded by the virus. The ability to reproduce the viral nucleic acid is not required, but is included, by this definition.

Typically, not every virus particle in a preparation is infectious. For example, particles can be damaged in preparation of the virus, thereby not affecting total particle number but decreasing the number of particles capable of infection. Furthermore, empty capsids or instability of the virus extracellularly can also contribute to the decrease in infectivity. The range of non-infectious particles to infectious particles in viral preparations can range from 1:1 to greater than 100:1. However, even non-infectious viruses can cause cytological changes or damage to exposed cells. Thus, it is advantageous to have an accurate measure of the number of infectious particles in a population so as to minimize the number of non-infectious viral particles to which cells are exposed.

Virtually any virus can be quantitated, or titered, by the methods of the instant invention, including DNA viruses, RNA viruses, replication competent viruses, replication incompetent viruses, recombinant viruses, viruses carrying transgenes, etc. Preferably, the virus can infect cells in culture. Some example of viruses amenable to this technique include, but are not limited to, adenovirus, adeno-associated virus, retrovirus, herpes simplex virus, parvovirus, Epstein Barr virus, rhinotracheitis virus, parainfluenza virus, bovine diarrhea virus, sindbis virus, baculovirus, pseudorabies virus, varicella-zoster virus, cytomegalovirus, HIV, hepatitis A, B, and C viruses, and vaccinia.

In some embodiments of the invention, infectivity is measured by antibodies directed against a polypeptide expressed by the virus. The polypeptide may be a structural viral polypeptide, a regulatory polypeptide, a polypeptide such as a polymerase, and so on. In some embodiments of the invention, the polypeptide is preferably expressed by an exogenous gene incorporated into the virus, such as a reporter gene. Some examples of reporter genes include β-galactosidase and chloramphenicol transacetylase (CAT). In further embodiments of the invention, the reporter gene is detected by antibodies directed against a product of the action of the reporter gene, such as the action of an enzyme on a substrate. In other embodiments of the invention, the exogenous gene is a transgene intended for therapeutic use. Some examples include but are not limited to tumor suppressor genes, including p53 or retinoblastoma (RB); interleukins, including IL-2, IL-4, and IL-10; interferons, including alpha-, beta-, and gamma interferon; other cytokines; thymidine kinase; growth factors, including GCSF and growth hormone; Factor VIII; adenosine deaminase, and so on. Typically production of polypeptide encoded by a transgene will be measured by an antibody directed against the polypeptide.

Antibodies used for detection can be polyclonal, monoclonal, or include mixtures of such antibodies. Typically, the detection is done directly by using a fluorescein-conjugated antibody directed against the viral polypeptide. However, indirect assays are also possible, in which the antibody directed against the viral polypeptide is then reacted with a fluorescein-labeled antibody. Any fluorescent label compatible with flow cytometry can be used.

To perform the assay of the invention, typically, the total number of virus particles in a viral preparation is first measured by any of a number of traditional techniques. For example, an aliquot of a virus preparation can be prepared in a buffer containing 0.1% sodium dodecyl sulfate (SDS), after which the optical absorbance is measured at 260 nm (Maizel et al. Virology **36:115-125** (1968)). Total particle counts can also be obtained by preparing a sample of the viral preparation for electron microscopy, and simply counting the number of particles. A further technique for particle enumeration can include the use of anion-exchange chromatography (Huyghe et. al. Human Gene Therapy **6:1403-1416** (1995)).

Cells are then infected with dilutions of the viral preparations at total particle number to cell number ratios no higher than about 100:1, typically less than about 10:1, preferably less than about 5:1, more preferably less than about 1:1. In some embodiments the ratio is as low as about 0.1:1. Typically, at least one infection will be performed, although in some embodiments at least two parallel infections are performed at different particle to cell ratios. The cells used are typically known to be sensitive to infection by the virus. It is not required that the cells support replication by the virus, but the infection is performed under conditions that allow expression of the viral polypeptide to be detected.

The total volume of a virus preparation used to infect cells in culture is typically determined by the skilled artisan by taking into account such factors as the total number of cells to be infected, the particle concentration of the virus preparation, and the volume of the vessel in which the infection is performed. Preferably, the particle concentration of virus used to infect cells in the infection mixture is at least about 10⁵ particles per ml, more preferably at least about 10⁶ particles per ml, most preferably about 10⁷ particles per ml. The viral preparations typically are prepared under conditions favorable to stability of the virus. Conditions for infection and, optionally, culture after infection will depend on the particular virus and the viral or reporter gene used for detection. The term "culture" as used herein refers to any form of cell culture in which the minimum requirements are provided to the cells to enable continued survival for the period of interest. Thus, for example, culture can refer to preparation of a cell suspension in a suitable buffer, such as phosphate buffered saline or an incomplete growth medium, for a period of minutes or hours, or can refer cells adhering to culture dishes for minutes to days to weeks in the presence of a suitable complete growth medium. Typically, sufficient time in culture is provided for expression of the desired viral polypeptide, but preferably not enough time is provided for propagation of the infecting virus which results in further infection of cells. Thus, it is preferable that only "one round" of infection occur in these cells. In some embodiments, the length of time allowed "in culture" will be less than 1 hour to several hours. In other preferred embodiments, the length of time will be 1 to 5 days.

Typically, cells are infected under conditions favoring adsorption of the virus to the cells, although less optimal conditions can be used in some embodiments. Typically, viruses are allowed to adsorb to cells for 1-12 hours. In some embodiments, the cells are infected in a concentrated suspension with concentrated virus, to enhance the rate of infection or the number of infected cells, then diluted to a concentration more favorable for cell or viral growth. In some embodiments of the invention, it can be desirable to wash infected cells cultures to remove unabsorbed virus or components of the medium used for infection, or to expose the infected cells to media or growth conditions more favorable to their survival.

After sufficient time has elapsed to allow expression of the viral polypeptide, the cells are typically prepared as a suspension of single cells. When the cells are infected as adherent cells in tissue culture, the cultures are typically treated with a dissociating agent such as trypsin to detach the cells from the substratum. Mechanical means can also be used to detach cells, such as scraping. Cells are then collected by centrifugation and prepared in a buffer, such as incomplete or complete growth medium, for reaction with the detection reagents. Typically cells are "fixed" for immunostaining by any of a number of standard techniques. A review of the commonly used fixation techniques is provided by Bauer and Jacobberger, Methods in Cell Biology **41:351-376** (1994)), hereby incorporated by reference in its entirety for all purposes. When the polypeptide is detected by its activity, fluorescent reagents can be introduced into cells to allow detection of the activity, such as a fluorescein labeled substrate for an enzyme.

Infected cell populations are then subjected to analysis by standard flow cytometry, such as by the methods disclosed by Shapiro, Practical Flow Cytometry, 3rd ed., John Wiley and Sons (1994). The term "FACS" is sometimes used to refer to flow cytometry, although cell sorting is not required to practice the instant invention. Typically, a minimum of about 10,000 events is acquired in the analysis. Dead cells are typically excluded from the analysis either by forward/side scatter gating or PI labelling and setting of electronic windows on the PI negative fraction. A variety of commercial software packages are available to aid in preparation and analysis of the data, such as CellQuest™.

The following example is intended to illustrate but not limit the invention in any way.

### EXAMPLE

In this example, ACNRB, a recombinant, replicative-defective adenovirus was titered by TCID₅₀ and by the low particle number to cell number ratio (low ratio) method of the present invention. The exemplary virus used essentially comprised the adenovirus vector backbone disclosed by Wills et al. (Cancer Gene Therapy **2:191-197** (1995)) with full-length retinoblastoma cDNA inserted into the vector.

Total particle number was obtained by the "SDS/OD₂₆₀" method and anion exchange chromatography methods described above. In both assays the measured total particle concentration was 1.0 X 10¹²/ml.

Infectious particles were titered by TCID₅₀ assay as described by Huyghe et al. (Human Gene Therapy **6:1403-1416** (1995)). In brief, 293 cells were plated into a 96-well microtiter plate: 100µl of 5x10⁵ cells/ml for each well in complete MEM (10% bovine calf serum; 1% glutamine) media (GIBCO BRL). In a separate plate, a 250-µl aliquot of virus sample diluted 1:10⁶ was added to the first column and was serially diluted two-fold across the plate. Seven rows were used for samples. One row was used for a negative control. A 100-µl aliquot of each well was transferred to its identical position in the 293 seeded plate and allowed to incubate a 37°C in a humidified air/7% CO₂ incubator for 2 days. The media was then decanted by inversion and the cells fixed with 50% acetone/50% methanol. After washing with PBS, the fixed cells were incubated for 45 minutes with a FITC-labeled anti-Ad5 antibody (Chemicon International #5016) prepared according to the kit instructions. After washing with PBS, the plate was examined under a fluorescent microscope (490 mm excitation, 520 mm emission) and scored for the presence of label. The titer was determined using the Titerprint Analysis program (Lynn, Biotechniqses **12:880-881** (1992)).

The low ratio assay was performed as follows. 1 X 10⁶ 293 cells (human embryonic kidney cells, ATCC CRL 1573) were seeded per well on 4 6-well dishes. The final volume per well was 1 ml. After about 2 hr, the medium (Dulbecco's modified Eagle's medium (DME high glucose) containing 4500 mg/ml D-glucose, supplemented with 5% defined, iron-supplemented bovine calf serum, 2mM L-glutamine, and 1 mM sodium pyruvate) in each well was aspirated and replaced with 1.1 ml of medium (without serum) containing diluted virus. Adsorption was allowed to occur for 60 minutes, after which an additional 2ml of virus-free medium was added to each well. After about 42 hr, the infected cells cultures were processed for flow cytometry analysis.

The cells were detached from the plastic substratum with a trypsin-EDTA solution (GIBCO-BRL). Detached cells were collected from each well and centrifuged at about 200 x g for 10 minutes at room temperature. The supernatants were removed and the cells washed in Dulbecco's phosphate buffered saline (D-PBS) without calcium or magnesium salts. Pelleted cells were then resuspended in 2 ml cold acetone:methanol (1:1) fixative, then held on ice for 15 minutes. 7 ml D-PBS without calcium or magnesium salts was added to each tube, after which the cells were resuspended in D-PBS with 1% (v/v) calf serum. After repeating these last two steps, cells were resuspended in 50µl D-PBS with 1% calf serum. 70 µl anti-adenovirus antibody conjugated with FITC (Chemicon #5016) in 2.0 ml D-PBS was added to each tube. The samples were incubated at 37°C for about 50 minutes. The samples were then transferred to flow cytometry analysis tubes, diluted slightly with 0.5 ml D-PBS, and analyzed by flow cytometry. A Becton-Dickinson FACScan™ Flow Cytometer System, PN 34011570, 12-00189-01 with FACStation (MAC QUADRA 650 computer, monitor, and printer) was used with CellQuest™ Software.

The results are shown in Table 1. By the traditional TCID₅₀ assay, the total particle number to infectious unit ratio was 63:1. As is evident in the table, as the total particle number to cell number ratio decreased, the calculated total particle number: infectious unit ratio also decreased to as low as 12:1, thereby providing a value for infectious titer that was about 5-fold higher than the traditional assay. Thus, this low ratio assay provides an unexpectedly better (i.e. much more accurate) enumeration of the number of infectious particles in a viral preparation than traditional methods for titration. The consequences of such accurate measurements proved by the instant invention are especially important in calculating the effective doses of recombinant viruses for therapeutic use.

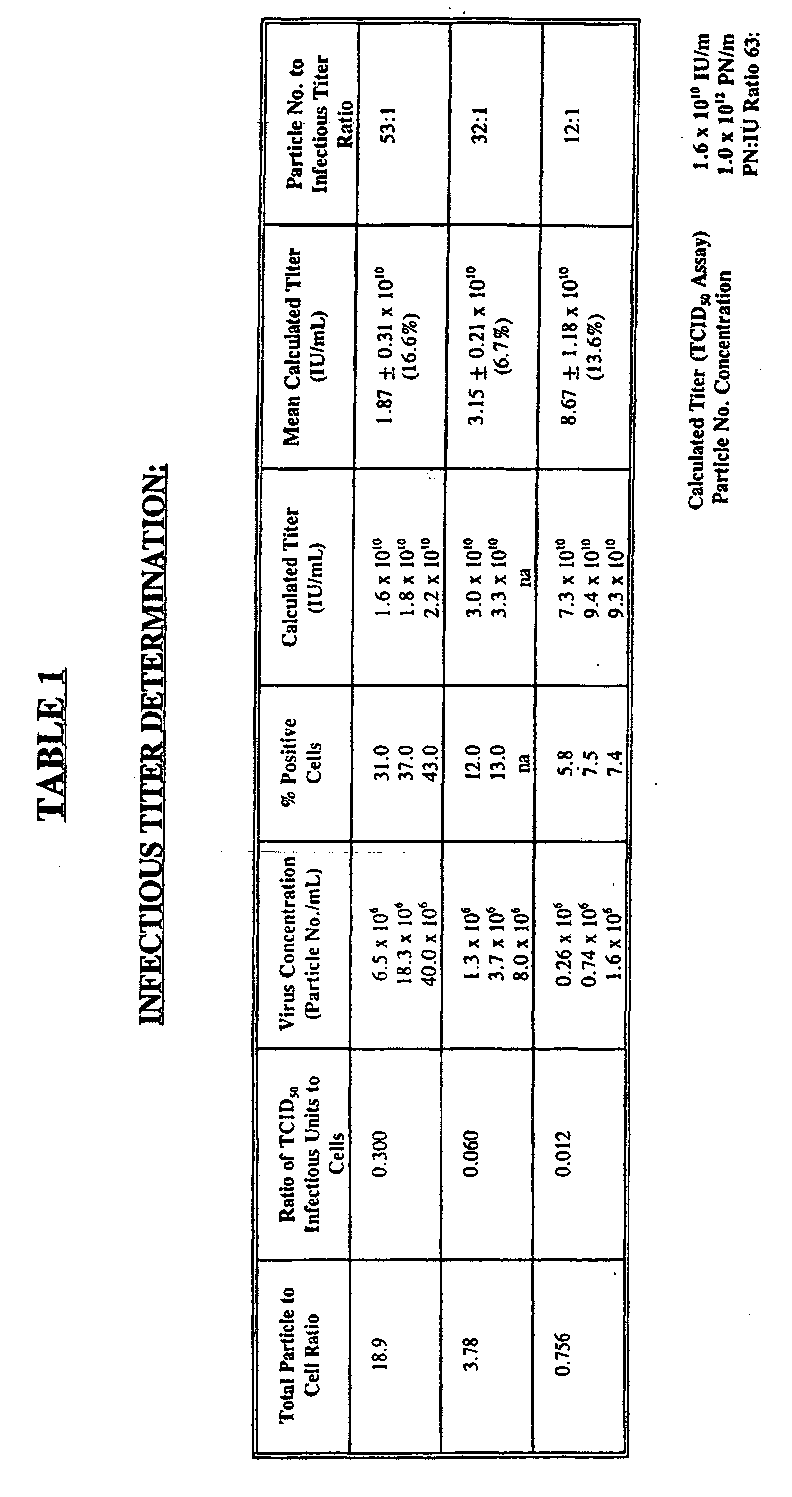

## Claims

1. A method for determining the number of infectious virus particles in a population of virus particles comprising:
i) infecting cells in a cell population at a total particle number to cell number ratio of less than about 100:1 to about 0.1:1 to generate infected cells wherein the cells are cultured after infection for a length of time that is sufficient for expression of a polypeptide expressed by the virus but is not enough time for propagation of the infecting virus to result in further infection of cells;
ii) reacting a polypeptide expressed by the virus in infected cells with an antibody labeled with a fluorescent tag, the antibody having specificity for a polypeptide expressed by the virus; and
iii) measuring immunofluorescence in the product of step (ii) by flow cytometry to determine the number of infected cells, thereby determining the number of infectious virus particles.

2. The method of claim 1, wherein the virus is adenovirus.

3. The method of claim 2, wherein the viral polypeptide is hexon.

4. The method of claim 1, wherein the virus is a recombinant virus.

5. The method of claim 4, wherein the viral polypeptide is encoded by an exogenous gene.

6. The method of claim 5, wherein the exogenous gene is a reporter gene.

7. The method of claim 5, wherein the exogenous gene is p53.

8. The method of claim 5, wherein the exogenous gene is retinoblastoma.

9. The method of claim 1, wherein the antibody is a mixture of antibodies.

10. The method of claim 1, wherein the antibody is polyclonal.

11. The method of claim 1, wherein the antibody is monoclonal.

12. The method of claim 4, wherein the recombinant virus is replication defective.

13. The method of claim 1, wherein the ratio is less than about 10:1 to about 0.1:1.

14. The method of claim 1, wherein the ratio is less than about 5:1 to about 0.1:1.

15. The method of claim 1, wherein the ratio is about 0.1:1.

## Patentansprüche

1. Verfahren zur Bestimmung der Anzahl von infektiösen Viruspartikeln in einer Population von Viruspartikeln, Schritte umfassend, bei denen man:
i) Zellen in einer Zellpopulation mit einem Partikelgesamtzahl/Zellzahl-Verhältnis von weniger als ungefähr 100 : 1 bis ungefähr 0,1 : 1 infiziert, um infizierte Zellen zu erzeugen, wobei die Zellen nach Infektion für eine Zeitdauer kultiviert werden, die für die Expression eines Polypeptids, welches durch das Virus exprimiert wird, ausreicht, aber nicht genug Zeit für eine Vermehrung des infizierenden Virus, welche zur weiteren Infektion von Zellen führen würde;
ii) ein Polypeptid, welches von dem Virus in den infizierten Zellen exprimiert wird, mit einem durch einen Fluoreszenzmarker markierten Antikörper umsetzt, wobei der Antikörper Spezifität für ein durch das Virus exprimiertes Polypeptid hat; und
iii) die Immunofluoreszenz in dem Produkt aus Schritt (ii) durch Durchflusscytometrie misst, um die Anzahl der infizierten Zellen und dadurch die Anzahl von infektiösen Viruspartikeln zu bestimmen.

2. Verfahren nach Anspruch 1, in dem das Virus Adenovirus ist.

3. Verfahren nach Anspruch 2, in dem das virale Polypeptid Hexon ist.

4. Verfahren nach Anspruch 1, in dem das virus ein rekombinantes Virus ist.

5. Verfahren nach Anspruch 4, in dem das virale Polypeptid durch ein exogenes Gen kodiert ist.

6. Verfahren nach Anspruch 5, in dem das exogene Gen ein Reportergen ist.

7. Verfahren nach Anspruch 5, in dem das exogene Gen p53 ist.

8. Verfahren nach Anspruch 5, in dem das exogene Gen Retinoblastoma ist.

9. Verfahren nach Anspruch 1, in dem der Antikörper eine Mischung von Antikörpern ist.

10. Verfahren nach Anspruch 1, in dem der Antikörper polyklonal ist.

11. Verfahren nach Anspruch 1, in dem der Antikörper monoklonal ist.

12. Verfahren nach Anspruch 4, in dem das rekombinante Virus replikations-defizient ist.

13. Verfahren nach Anspruch 1, in dem das Verhältnis geringer als ungefähr 10 : 1 bis ungefähr 0,1 : 1 ist.

14. Verfahren nach Anspruch 1, in dem das Verhältnis geringer als ungefähr 5 : 1 bis ungefähr 0,1 : 1 ist.

15. Verfahren nach Anspruch 1, in dem das Verhältnis ungefähr 0,1 : 1 ist.

## Revendications

1. Procédé destiné à déterminer le nombre de particules virales infectieuses dans une population de particules virales comprenant :
i. l'infection de cellules dans une population cellulaire en utilisant un rapport nombre total de particules/ nombre de cellules de moins d'environ 100 :1 à environ 0,1 :1 pour obtenir des cellules infectées où les cellules sont cultivées après infection pour une durée qui est suffisante pour exprimer un polypeptide exprimé par le virus mais insuffisante pour la propagation des virus infectieux pour obtenir la surinfection des cellules.
ii. la réaction d'un polypeptide exprimé par le virus dans des cellules infectées avec un anticorps marqué avec un marqueur fluorescent, l'anticorps possédant une spécificité pour un polypeptide exprimé par le virus ; et
iii. la mesure de l'immunofluorescence dans le produit de l'étape (ii) par cytométrie de flux afin de déterminer le nombre de cellules infectées et par conséquent le nombre de particules virales infectieuses.

2. Méthode selon la revendication 1, dans laquelle le virus est un adénovirus.

3. Méthode selon la revendication 2, dans laquelle le polypeptide viral est un exon.

4. Méthode selon la revendication 1, dans laquelle le virus est un virus recombinant.

5. Méthode selon la revendication 4, dans laquelle le polypeptide viral est codé par un gène exogène.

6. Méthode selon la revendication 5, dans laquelle le gène exogène est un gène rapporteur.

7. Méthode selon la revendication 5, dans laquelle le gène exogène est p53.

8. Méthode selon la revendication 5, dans laquelle le gène exogène est un rétinoblastome.

9. Méthode selon la revendication 1, dans laquelle l'anticorps est un mélange d'anticorps.

10. Méthode selon la revendication 1, dans laquelle l'anticorps est polyclonal.

11. Méthode selon la revendication 1, dans laquelle l'anticorps est monoclonal.

12. Méthode selon la revendication 4, dans laquelle le virus recombinant est incapable de se répliquer.

13. Méthode selon la revendication 1, dans laquelle le rapport est inférieur à environ 10 :1 à environ 0,1 :1.

14. Méthode selon la revendication 1, dans laquelle le rapport est inférieur à environ 5 :1 à environ 0,1 :1.

15. Méthode selon la revendication 1, dans laquelle le rapport est d'environ 0,1 :1.
